# FASCICULE DE BREVET EUROPEEN

(11) **EP 0 581 704 B1**
(45) Date de publication et mention de la délivrance du brevet: **24.01.2001**
(21) Numéro de dépôt: 93420323.3
(22) Date de dépôt: 27.07.1993
(51) Int. Cl.: A61B 8/14, A61B 8/08

(54) **Procédé de détermination de la position d'un organe**
Verfahren zur Bestimmung der Lage eines Organs
Method for determining the position of an organ

(30) Priorité: 31.07.1992 FR 9209801; 27.11.1992 FR 9214594
(43) Date de publication de la demande: 02.02.1994
(73) Titulaire: UNIVERSITE JOSEPH FOURIER, F-38041 Grenoble Cédex (FR)
(72) Inventeur: Cinquin, Philippe, FR-38000 Grenoble (FR); Lavallée Stéphane, FR-38000 Grenoble (FR); Dubois Françis, FR-38240 Meylan (FR); Brunie Lionel, FR-38000 Grenoble (FR); Troccaz Jocelyne, FR-38320 Eybens (FR); Peria Olivier, FR-73100 Aix-les-Bains (FR); Mazier Bruno, FR-38000 Grenoble (FR)
(74) Mandataire: de Beaumont, Michel

(56) Documents cités:
- EP-A- 0 427 358
- WO-A-92/04862
- DE-A- 3 908 648
- DE-A- 4 021 102
- US-A- 4 618 978

## Description

La présente invention concerne le domaine de la chirurgie et de l'observation médicale et plus particulièrement des procédés et dispositifs de positionnement d'un outil thérapeutique ou diagnostique en fonction d'images spatiales, ces images spatiales pouvant être des images réalisées au préalable (images pré-opératoires) d'un organe d'un patient.

Dans la présente demande de brevet, on entend par "outil" tout moyen d'action thérapeutique ou diagnostique sur un patient. L'outil peut par exemple être un dispositif propre à insérer une vis dans un os d'un patient, un trocart destiné à réaliser une ponction ou simplement à acheminer une fibre optique, un appareil d'émission de rayons destinés à agir sur une tumeur, ou encore un appareil d'imagerie médicale tel qu'une caméra de gamma-scintigraphie, un appareil de tomographie par émission de positrons (TEP), ou un appareil de magnétoencéphalographie (MEG).

En d'autres termes, un objectif est de retrouver, durant un geste médico-chirurgical, les informations morphologiques que des examens tridimensionnels préalables avaient permis d'obtenir. Cette situation est très fréquente cliniquement. Nous en donnerons ci-après deux exemples.

### 1° Radiothérapie

La radiothérapie est l'acte consistant à projeter sur une zone donnée d'un patient ou d'un organe d'un patient un flux de rayonnement de façon à détruire des tumeurs existant dans cet organe. De tels traitements doivent généralement être effectués de façon périodique et répétée. Il faut donc à chaque intervention replacer la source de rayonnement par rapport au patient pour irradier la zone choisie et autant que possible de la façon la plus spécifique possible pour éviter d'irradier d'autres zones du patient sur lesquelles le flux de rayonnement aurait un effet néfaste.

Le diagnostic initial a généralement été effectué par un scanner à rayons X ou par imagerie par résonance magnétique (IRM) qui permettent de visualiser la cible et les obstacles et donc de définir une stratégie thérapeutique optimale. Toute la difficulté est de repositionner le patient quand on veut lui faire subir le traitement. La méthode actuellement couramment utilisée consiste à simuler l'intervention en remplaçant l'accélérateur linéaire (la source de rayonnement) par un tube à rayons X. On obtient ainsi deux radiographies (face et profil) du patient que l'on compare (visuellement) aux informations tridimensionnelles préalables provenant du scanner X ou de l'IRM. On déplace le patient et l'on refait des clichés jusqu'à ce que l'on soit satisfait de son positionnement. Un trait lumineux matérialise alors une marque sur la peau du patient et l'on inscrit cette marque à même la peau du patient à l'aide d'un marqueur. Lors de la séance d'irradiation, on déplacera le patient jusqu'à ce que ces marques cutanées coïncident avec des faisceaux lumineux identiques pour l'accélérateur linéaire et pour le système radiologique de simulation. Ce procédé classique présente de nombreux inconvénients. L'un deux est qu'il oblige à déplacer le patient sur la table de soin pour lui faire prendre une position souhaitée, ce qui n'est pas toujours facile et qui peut amener le patient à prendre des positions inconfortables ou à être dans un état crispé tel qu'il ne pourra pas maintenir sa position. Il faut souligner que le positionnement du patient doit être aussi précis que possible. Par exemple dans le cas de la phase finale de la radiothérapie de la prostate, où seul cet organe, inclus dans une sphère de 4 cm de diamètre, est visé, on est amené avec les procédés classiques à irradier avec des champs de 10 cm de diamètre pour être certain que la prostate est correctement atteinte. Ceci n'est bien entendu pas sans inconvénient pour les zones environnantes.

### 2° Orthopédie

Il s'agit ici d'aider à l'introduction de matériel (par exemple une vis) dans le corps humain (couramment dans un os) selon une trajectoire linéaire. La forme de l'os est classiquement étudiée par un moyen d'imagerie tridimensionnel (scanner X ou IRM) et la stratégie d'intervention est ensuite déterminée en fonction de cette image, notamment en ce qui concerne la direction exacte dans laquelle devra être insérée l'outil pour atteindre exactement l'emplacement souhaité sans passer par des zones où il risque d'être destructif.

Classiquement, pendant la phase opératoire, on utilise des procédés radiographiques pour contrôler l'introduction de l'outil. L'inconvénient majeur est que, dans les conditions habituelles, on ne peut pas réaliser simultanément des images face et profil de la cible et du matériel. On ne peut donc utiliser ce procédé que comme moyen de vérification a posteriori de la qualité du positionnement du matériel (la progression n'étant suivie en temps réel que sur une projection). D'autre part, cette vérification ne peut être réalisée de manière quantitative. L'interprétation est laissée à l'opérateur. Les études actuelles montrent qu'avec ces techniques, il existe pratiquement 10 % des cas dans lesquels le positionnement n'est pas idéal.

Le document EP-A-0 427 358 divulgue un procédé de détermination de la position d'un organe d'un patient à l'aide d'un dispositif de formation d'image dans une enceinte opératoire après avoir réalisé une image tridimensionnelle de l'organe du patient avec un deuxième dispositif de formation d'image dans une enceinte pré-opératoire, le procédé comprennant la fusion des images des deux dispositifs en utilisant des points de référence fixés sur le patient.

Le document WO-A-92/04 862 divulgue un procédé similaire dans lequel les images d'une sonde écographique montée sur un bras articulé dans un premier référentiel sont fusionnées avec des images biomagnetiques obtenues dans un deuxième référentiel.

On a ensuite proposé pour améliorer l'utilisation per-opératoire du système radiologique de mettre en oeuvre des techniques de fusion d'images tridimensionnelle et bidimensionnelle (S. Lavallée et al., Matching 3D smooth surfaces with their 2D projections using 3D distance maps, SPIE 1570, P. 322-336, 1991). Toutefois, ce procédé est coûteux car il nécessite d'utiliser un système radiologique de bonne qualité capable d'étudier la cible sur plusieurs incidences et dont le signal puisse être numérisé. Pour éviter les inconvénients des systèmes radiologiques, plusieurs équipes médicales ont proposé de réaliser des interventions sous scanner ou IRM. Il est alors facile de réaliser la comparaison entre les images pré-opératoire et per-opératoire effectuées par le même moyen. Toutefois, ces approches présentent de nombreux inconvénients du fait qu'elles imposent la présence simultanée d'outils d'imagerie sophistiqués et d'instruments opératoires. Parmi ces inconvénients, on peut notamment citer :
- contraintes chirurgicales (asepsie...),
- immobilisation longue (pendant toute une opération) d'un matériel coûteux (scanner X ou IRM) alors que l'on tente au contraire d'utiliser en continu ces appareils pour des tâches d'observation pour assurer leur rentabilité,
- nécessité de prévoir un matériel chirurgical spécifique (dans le cas de l'IRM, ce matériel doit être non générateur d'artéfacts et en particulier non-ferromagnétique) ; ceci amène par exemple à réaliser des outils en titane qui sont particulièrement coûteux,
- la géométrie même des appareils d'imagerie (tunnel de diamètre étroit) rend l'introduction de matériel chirurgical difficile.

Toutefois, l'essor de ces techniques de fusion d'images tridimensionnelles malgré toutes les difficultés qui ont été résumées ci-dessus montre l'intérêt de la communauté médicale pour tout moyen susceptible d'améliorer la précision de l'introduction de matériel médico-chirurgical.

Un objet de la présente invention est de prévoir un procédé simple et peu coûteux pour réaliser une série d'images per-opératoires d'un organe permettant de positionner un outil d'une façon déterminée par rapport à une série d'images préopératoires tridimensionnelles.

Pour atteindre cet objet, la présente invention prévoit d'utiliser, pour réaliser les images per-opératoire une sonde écographique fournissant une image morphologique tridimensionnelle de points de surface de l'organe ou d'une région cutanée fixe par rapport à l'organe. Après quoi, cette image de points de surface est combinée (fusionnée) avec l'image tridimensionnelle fonctionnelle pré-opératoire qui contient aussi des informations de localisation des points de surface (de l'organe ou d'une région cutanée).

Pour obtenir une image morphologique tridimensionnelle de points de surface de l'organe, la présente invention prévoit d'utiliser des sondes échographiques. L'image morphologique tridimensionnelle de points de surface d'une région cutanée fixe par rapport à l'organe peut être, par exemple, une image d'une portion de la tête qui est fixe par rapport au cerveau.

En outre, la présente invention prévoit d'aligner le référentiel de l'outil par rapport au référentiel de l'organe lui-même déterminé par l'image pré-opératoire.

Un deuxième objet de la présente invention est de réaliser une fusion d'image entre l'image de la sonde écographique fournissant comme cela a été indiqué une image morphologique tridimensionnelle de points de surface liés à un organe, et l'image d'un troisième appareil placé dans le même site opératoire tel qu'une caméra de gamma-scintigraphie, un appareil de tomographie par émission de positrons (TEP) ou un appareil de magnétoencéphalographie (MEG) fournissant des informations fonctionnelles sur des zones spécifiques de cet organe, d'où il résulte qu'il est possible de positionner ces zones spécifiques de l'organe.

Pour atteindre ce deuxième objet, la présente invention prévoit de repérer au préalable une position initiale du premier dispositif et du deuxième appareil en leur faisant viser une même cible visible pour chacun d'eux (par exemple en ultrasons et en rayons gamma dans le cas d'une sonde échographique et d'une caméra de gamma-scintigraphie).

L'une des originalités de l'invention réside dans l'utilisation d'un dispositif ne fournissant pas d'information fonctionnelle mais seulement des images de points de surface pour servir d'intermédiaire permettant des repérages de référentiels. En particulier, l'idée d'utiliser une sonde échographique pour réaliser cet alignement de référentiels constitue l'un des aspects de la présente invention étant donné que, a priori, une image échographique donne une information moins riche que les procédés de type IRM ou scanner. En effet, l'échographie fournit habituellement une série de coupes planes et indépendantes d'un organe, et non un volume structuré en série de coupes parallèles.

### Rappels sur l'échographie

L'échographie est une modalité d'imagerie qui a pris son essor dans les années 1970. Un transducteur (cristal piézoélectrique) émet des ultrasons (à des fréquences de plusieurs mégahertz) qui se propagent dans le corps humain, mais sont susceptibles de réflexion lorsqu'ils abordent des interfaces où l'impédance acoustique du milieu subit de fortes variations (typiquement, une interface eau/graisse). Le même transducteur peut être utilisé durant une période brève comme émetteur d'ultrasons, et, pendant une période généralement plus longue, comme récepteur des ultrasons réfléchis par le corps humain. Il est alors possible de mesurer à la fois l'intervalle temporel entre émission et réception (ce qui permet, moyennant des hypothèses sur la célérité des ultrasons dans le milieu considéré, de déterminer la localisation de la source échogène) et l'intensité de l'écho (ce qui donne des informations sur la nature du point échogène).

Le mode de fonctionnement le plus simple consiste à n'émettre et à ne recueillir les ultrasons que dans une direction (mode A). On obtient alors un signal, variable en fonction du temps si les structures échogènes sont mobiles. Ce mode de fonctionnement a été le premier utilisé en médecine, particulièrement dans les applications cardio-vasculaires (où il permettait d'évaluer la mobilité des valves cardiaques, par exemple).

Il est également possible d'émettre et de recueillir les ultrasons dans une portion plane (généralement trapézoïdale) de l'espace (mode B). Ceci peut être réalisé par la juxtaposition sur une barrette linéaire d'une série de transducteurs mono-dimensionnels fixes, ou par la mise en rotation (mécanique ou électronique), dans un plan, autour d'un point fixe, d'un transducteur mono-dimensionnel. Cette modalité d'imagerie s'est révélée très précieuse pour l'étude des "organes mous", en particulier dans les domaines gynécologiques et obstétricaux, ainsi qu'en gastro-entérologie.

Il y a en outre de nombreuses situations cliniques où ce sont les échographies qui fournissent les informations les plus intéressantes (en particulier en gastro-entérologie, en gynéco-obstétrique et en cardiologie). Les échographes sont aussi un moyen précieux de guidage d'interventions médicales (ils permettent par exemple de contrôler l'introduction d'aiguilles de ponction à l'intérieur du corps humain).

D'autre part, à qualité d'information équivalente, l'échographie présente par rapport au scanner X et à l'IRM les avantages suivants :
- environ 10 fois moins cher
- l'émetteur est réalisable sous forme d'une sonde que l'on plaque contre le corps mais qui est légère et facile à porter. Par contre, les appareils de type scanner X et IRM sont des appareils extrêmement encombrants qui occupent un volume important dans une salle d'examen,
- par rapport au scanner X, l'échographie, comme l'IRM, présente l'avantage d'une parfaite innocuité.

Mais surtout, l'avantage essentiel de l'échographie est qu'il s'agit d'une modalité d'imagerie morphologique de bonne qualité simple d'emploi et peu coûteuse.

En outre, les préjugés habituels écartent les méthodes d'échographie dès qu'il s'agit d'analyses orthopédiques, c'est-à-dire d'analyses concernant des os. En effet, dans les conditions habituelles, les ultrasons ne traversent pas les os qui les réfléchissent spéculairement. Il est donc impossible d'étudier l'intérieur des os par des ultrasons. L'étude de la surface des os est cependant possible bien que rendue délicate par le caractère spéculaire de la réflexion des ultrasons. La réflexion ne se faisant quasiment que dans la direction donnée par la loi de Descartes, chaque échographie d'un os donnera des images très pauvres en informations : seules seront vues des portions de contour d'os dont la normale est parallèle à la direction des ultrasons.

Toutefois, pour l'application envisagée ici, il suffit comme on le montrera ci-après de recueillir une information sur un point de la surface d'un os à chaque prise de vue échographique pour pouvoir mettre en oeuvre le procédé selon l'invention. L'échographie s'applique donc alors également à des applications orthopédiques.

Plus précisément, la présente invention prévoit un procédé de détermination de la position d'un organe d'un patient par rapport à au moins deux dispositifs de formation d'image comme cela est défini dans la revendication 1.

Des modes de réalisation de la présente invention sont définis dans les sous-revendications.

Ces objets, caractéristiques et avantages ainsi que d'autres de la présente invention seront exposés en détail dans la description suivante de modes de réalisation particuliers faite en relation avec les figures jointes parmi lesquelles :
la figure 1 illustre un exemple de mire échographique utilisée selon la présente invention ; et
la figure 2 représente un exemple de mire mixte échographie/gamma-scintigraphie utilisée selon la présente invention.

Comme cela a été rappelé précédemment, dans plusieurs actes médicaux, diagnostiques ou thérapeutiques, il est nécessaire de fusionner (c'est-à-dire de faire se correspondre de façon déterminée) un premier référentiel dans lequel un examen préalable, pré-opératoire, a permis d'étudier une partie de l'organisme et de déterminer une stratégie opératoire, et un deuxième référentiel correspondant au système dans lequel est réalisé l'acte opératoire. Les méthodes actuelles sont essentiellement des méthodes de mise en place de marqueurs visibles aussi bien lors de l'acte opératoire que lors de l'examen pré-opératoire et comme on l'a indiqué précédemment sont souvent imprécises et difficiles d'emploi.

L'examen pré-opératoire doit permettre d'identifier des objets anatomiques (une vertèbre, une prostate, le cerveau...) dont la forme ou la forme de l'enveloppe cutanée (la peau du crâne pour le cerveau) servira de référence pour la fusion des référentiels. Ainsi, on se place dans le cas où les référentiels sont "fusionnés" en fonction de l'organe lui-même ou de son enveloppe cutanée. Diverses techniques de traitement d'image permettent de réaliser ces opérations en passant par une étape dite de segmentation tridimensionnelle (F. Leitner, I. Marque, S. Lavallée, P. Cinquin, Dynamic segmentation : finding the edges with spline snakes, Proc. Int. Conf. on Curves and Surfaces, Chamonix, Academic Press, 279-284, 1991).

### Utilisation de la sonde échographique

La présente invention vise un procédé dans lequel l'image opératoire résulte d'un nuage de points obtenus par examen échographique de la zone concernée qui permet de visualiser des objets préalablement segmentés.

La difficulté réside dans la conception d'un protocole qui permettra de relier les coordonnées des points observés en échographie au référentiel per-opératoire. Pour ce faire, la position de la sonde échographique doit pouvoir être repérée dans le référentiel per-opératoire.

Selon un premier mode de mise en oeuvre de l'invention, on peut prévoir sur la sonde elle-même des points de repère détectables par un capteur adéquat (par exemple des diodes photoluminescentes, des émetteurs d'ultrasons) fixé rigidement par rapport au référentiel opératoire.

Selon un autre mode de réalisation préféré de la présente invention, la sonde est fixée rigidement à l'extrémité d'un bras articulé de robot. On détermine alors d'une part la position de la sonde par rapport au bras articulé, d'autre part la position du référentiel du bras articulé par rapport au référentiel opératoire.

### a) Détermination de la position relative de la sonde par rapport au bras articulé.

Pour réaliser cette détermination, la présente invention propose de déterminer la position de la sonde par rapport à une mire de calibrage qui permet de visualiser en échographie des points de repère dont la distribution spatiale est fixe. Ces points de repère sont étudiés en échographie et leur position spatiale réelle est comparée avec celle que fournit le changeur de coordonnées du bras du robot pour une position théorique de la sonde. On peut alors par une technique des moindres carrés non-linéaires identifier des paramètres de rotation et de translation qui caractérisent le passage du référentiel de la sonde à celui du bras.

La sonde étant fixée rigidement à l'extrémité du bras, il s'agit de trouver la transformation qui lie le repère de la sonde au repère du bras articulé. Pour cela, on étudie trois points de référence d'une mire qui doivent pouvoir être étudiés dans au moins deux positions quelconques.

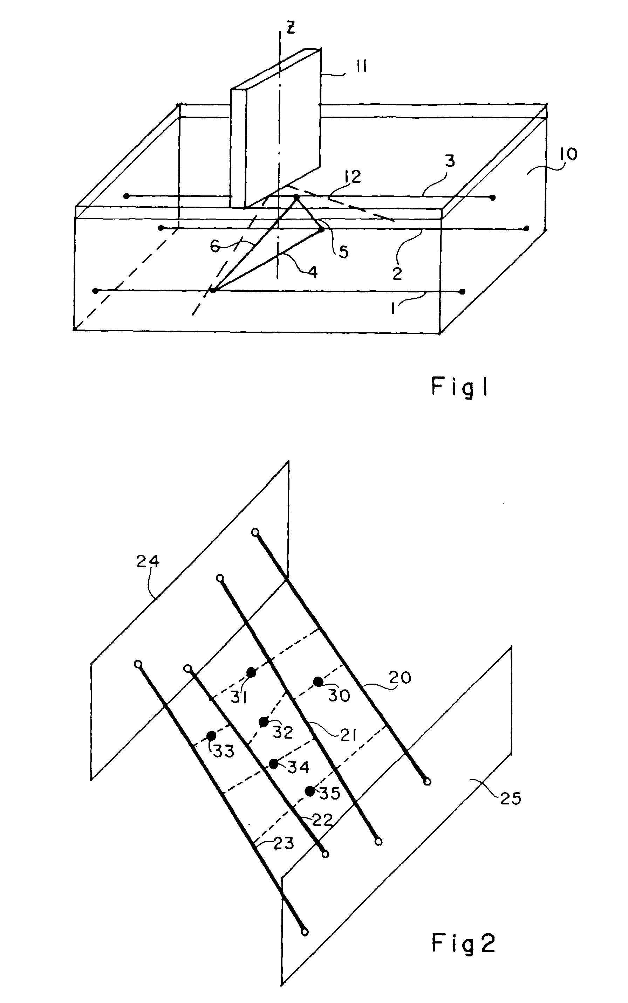

Un mode de réalisation d'une mire selon la présente invention est illustré en figure 1. Cette mire comprend, dans un milieu transmettant les ultrasons, par exemple une cuve à eau 10, trois fils 1, 2 et 3 tendus entre deux plans. Trois autres fils, 4, 5 et 6 relient deux à deux les trois fils 1, 2 et 3 et forment un triangle. Le triangle peut être fabriqué par un fil fin en un matériau échogène, par exemple un fil de Nylon. Ensuite, au moyen du manipulateur d'un bras articulé (non représenté), on dispose une sonde 11 pour que son faisceau plan 12 soit coplanaire avec le plan des trois fils 4, 5 et 6. Lorsque la sonde est dans le plan du triangle, on visualise alors parfaitement ce triangle dont on extrait le sommet en calculant l'intersection de ses arêtes. A partir de cette position de la sonde 11, on fait tourner la sonde de 180° autour de l'axe Z compris dans le plan de l'image échographique. Ceci permet d'identifier les paramètres de rotation.

On déplace la mire de calibrage vers une autre position quelconque et on répète l'opération de saisie des points de la mire dans deux positions du bras articulé porteur de la sonde échographique tournées l'une par rapport à l'autre de 180°. On dispose alors de toutes les données nécessaires pour mettre en oeuvre une méthode de calibrage classique, telle que décrite par exemple par Y. C. Shiu et al., Finding the mounting position by solving a homogeneous transform equation of form AX=XB, CH2413-3/87/0000/1666 1987 IEEE.

### b) Détermination de la position du référentiel du bras articulé et de la sonde par rapport au référentiel de l'outil opératoire.

L'outil opératoire, par exemple un guide d'introduction d'une aiguille, peut avoir été usiné conjointement avec le bras articulé porte-sonde. Dans ce cas, aucun problème de positionnement particulier ne se pose.

Par contre, si l'outil opératoire a été conçu indépendamment du bras articulé porte-sonde, il faut repérer mutuellement leurs référentiels.

Dans un premier exemple, l'outil opératoire est une caméra de gamma-scintigraphie ou un tomographe, et on utilise pour faire le repérage une mire mixte visible à la fois en échographie et en gamma-scintigraphie.

La figure 2 représente un exemple d'une telle mire constituée de quatre fils non-parallèles (20-23) tendus entre deux plans parallèles. Ces quatre fils sont des tubes minces tendus entre deux plaques de plexiglas 24, 25 et sont remplis d'un produit radioactif. La mire, une fois placée dans une cuve à eau, peut donc être vue à la fois en échographie et en scintigraphie. Sur chaque coupe scintigraphique, on détecte quatre sources radioactives et sur la sonde échographique on observe directement les intersections des quatre tiges 20, 21, 22 et 23 tendues entre les plaques de plexiglass 24 et 25 avec le plan échographique. A partir de ces données, on peut reconstituer les équations des quatre droites 20, 21, 22, 23 dans les repères d'échographie et de caméra scintigraphique.

Pour chaque paire de tiges 20, 21, 22, 23, on calcule ensuite un point virtuel 30-35 définissant le milieu du segment dont la longueur est la distance minimale entre deux tiges. On obtient ainsi un ensemble de six points dans le repère échographique et de six points dans le repère de la caméra. On peut ensuite appliquer une méthode de minimisation connue, par exemple celle décrite par K.S. Arun et al. dans IEEE Transactions on Pattern Analysis and Machine Intelligence, Vol. PAMI. 9 N° 5, pp. 698-700.

Dans un deuxième exemple, l'outil opératoire est un appareil de radiothérapie qui est souvent associé à des sources de faisceaux laser permettant de localiser un réflecteur en forme de coin à arêtes orthogonales. On utilise comme mire mixte une mire échographique, par exemple du type de celle de la figure 1, à laquelle est fixé, de façon bien déterminée par rapport aux trois fils 4, 5, 6, un coin réflecteur que l'on positionne, pour le calibrage initial, à l'intersection des faisceaux laser.

Selon un premier aspect de la présente invention, une fois la sonde échographique parfaitement positionnée par rapport au référentiel opératoire, on applique le procédé selon l'invention de prise d'un nuage de points image de la surface de l'organe concerné pour plusieurs positions de la sonde échographique de façon à déterminer la position de la sonde par rapport au référentiel de l'organe déterminé par une image tridimensionnelle pré-opératoire. On pourra alors adapter la position d'un outil repéré par rapport à la sonde à une stratégie déterminée lors de la phase pré-opératoire.

Selon un deuxième aspect de la présente invention, une fois la sonde échographique repérée par rapport à une mire mixte du type de celle de la figure 2, on repère une position initiale par rapport à cette mire mixte d'une caméra de gamma-scintigraphie ou de tomographie. La sonde échographique est ensuite déplacée de façon déterminée par rapport à cette position initiale, par exemple par un bras de robot codé. On connaît ainsi ensuite à tout instant avec précision la position mutuelle de la caméra et de la sonde. Il est donc possible de fusionner des images échographiques (morphologiques) et des images gamma-scintigraphiques ou tomographiques (fonctionnelles), ce qui fournit une aide précieuse au diagnostic. Il est en outre possible, selon le premier aspect de l'invention, de fusionner les images échographiques et une image morphologique tridimensionnelle résultant d'une analyse par un scanner X ou un appareil d'IRM. La combinaison de ces deux aspects de l'invention permet, les images échographiques servant d'intermédiaire, de fusionner l'image de gamma-scintigraphie et l'image morphologique tridimensionnelle résultant de l'analyse par un scanner X ou un appareil d'IRM.

Bien entendu, la présente invention est susceptible de nombreuses variantes qui apparaîtront à l'homme de l'art, notamment en ce qui concerne le choix des images échographiques à réaliser, et les modes d'obtention d'une corrélation entre l'image tridimensionnelle pré-opératoire et l'image d'un nuage de points obtenus par échographie d'un même organe.

Comme cela a été indiqué précédemment, l'organe que l'on veut repérer peut être fixe par rapport à une région cutanée du patient. C'est par exemple le cas du cerveau par rapport à la tête du patient.

### Rappels sur la Magnétoencéphalographie (MEG)

Il s'agit d'un appareil qui vise à étudier le fonctionnement du cerveau, en analysant le champ magnétique émis par celui-ci lors de diverses activités cérébrales. Ce champ est extrêmement faible (beaucoup plus faible que le champ magnétique terrestre). On introduit donc la tête du patient dans un dispositif constitué de plusieurs sondes capables de recueillir les champs à la surface du crâne. Le nombre d'électrodes utilisé est relativement limité (les appareils les plus performants semblent permettre d'utiliser 128 électrodes). Pour déterminer une carte d'intensité magnétique à l'intérieur du cerveau, on est donc dans des conditions beaucoup plus défavorables que, par exemple, celles de la reconstruction d'images tomodensitométriques à partir de projections radiographiques. En effet, d'une part le nombre de données disponibles est beaucoup plus faible (en tomodensitométrie, on disposera pour chaque coupe de plusieurs centaines de points de mesure par projection, et de plusieurs centaines de projection), d'autre part, la propagation des ondes électro-magnétiques est fortement variable selon les caractéristiques électro-physiologiques des tissus traversés. Le "problème inverse" à résoudre pour la MEG est donc beaucoup plus mal posé que celui de la tomodensitométrie. Ceci explique l'intérêt de l'injection dans ce problème de toute connaissance a priori disponible. Or, des examens réalisables avant la MEG donnent des informations potentiellement très importantes pour la reconstruction des images MEG. La tomodensitométrie permet par exemple de parfaitement identifier la boîte crânienne osseuse. Or, l'os est le barrage le plus important dans la diffusion des ondes électro-magnétiques émises par le cerveau. La quantification de son épaisseur et sa localisation précise sont donc des informations précieuses pour la MEG. Les structures ventriculaires, dont les caractéristiques électriques sont très particulières, sont d'autre part parfaitement identifiables. L'imagerie par résonance magnétique, quant à elle, permet de distinguer substances grise et blanche (aux caractéristiques électriques différentes), mais aussi de localiser les zones potentiellement émettrioes. L'examen en MEG peut en effet consister à enregistrer l'activité électrique du cerveau lors de tâches sensori-motrices ou conceptuelles bien précises, dont on sait qu'elles mettent en oeuvre des zones très bien connues du cerveau, localisables en IRM.

Tout ceci souligne l'intérêt d'une méthode facile et fiable pour repositionner le référentiel d'examens pré-opératoires tels que l'IRM ou le scanner, avant même de commencer à calculer des images de MEG. Prendre en compte les informations "pré-MEG" ainsi disponibles entraîne une amélioration considérable de la qualité des images de MEG.

## Revendications

1. Procédé de détermination de la position d'un organe d'un patient par rapport à au moins deux dispositifs de formation d'image, dans lequel
le premier dispositif de formation d'image est une sonde échographique placée dans une enceinte opératoire associée à un premier référentiel et fournit une image de points de la surface de l'organe ou d'une région cutanée du patient,
le deuxième dispositif de formation d'image est placé dans une enceinte pré-opératoire associée à un deuxième référentiel et fournit une image tridimensionnelle de la surface dudit organe et le cas échéant de la surface cutanée,
ce procédé comprenant les étapes suivantes :
déterminer la position du premier dispositif par rapport au premier référentiel ;
effectuer au moins une prise de vue avec le premier dispositif de façon à obtenir une première image correspondant à un nuage de points de la surface de l'organe ou de la région cutanée ;
prendre au moins une image tridimensionnelle comme deuxième image de l'organe et de sa surface ou de la surface de la région cutanée à l'aide du deuxième dispositif et identifier la forme de cette surface pour obtenir une référence ; et
fusionner les première et deuxième images en utilisant ladite référence, d'où il résulte que le premier référentiel est repéré par rapport au deuxième ;
où l'étape de détermination de la position du premier dispositif par rapport au premier référentiel comprend les étapes consistant à :
- fixer la sonde sur un bras de robot articulé codé,
- viser trois points d'une mire échographique, constituée de trois fils parallèles (1-3) tendus entre deux plans, trois autres fils (4-6) en un matériau réfléchissant les ultrasons étant disposés en triangle en appui sur les trois fils parallèles, l'ensemble étant noyé dans un milieu transmettant les ultrasons, tel qu'une cuve à eau,
- faire tourner la sonde de 180° autour de son axe de visée et viser à nouveau les trois points de la mire, et
- répéter les deux étapes précédentes pour une autre position de la mire.

2. Procédé selon la revendication 1, caractérisé en ce que le premier référentiel est le référentiel du support du patient.

3. Procédé de détermination de la position d'un organe selon la revendication 1, caractérisé en ce que la sonde échographique est susceptible de fournir à chaque prise de vue les distances dans un plan de visée entre cette sonde et l'interface dans ledit plan entre ledit organe et le milieu avoisinant, et en ce que l'étape de prise de vue consiste à déplacer la sonde et effectuer une prise de vue pour chacune de plusieurs positions déterminées de cette sonde de façon à obtenir pour chaque prise de vue la position par rapport à la sonde d'au moins un point de la surface de l'organe, d'où il résulte que l'on obtient une première image correspondant à un nuage de points de la surface de l'organe.

4. Procédé de détermination de la position d'un organe selon la revendication 1, caractérisé en ce qu'il consiste à :
- déterminer un axe d'action d'un outil opératoire en relation avec l'image pré-opératoire prise dans le deuxième référentiel,
- identifier cet axe d'action dans le premier référentiel, et
- positionner un outil selon cet axe d'action dans le premier référentiel.

5. Procédé selon la revendication 4, dans lequel l'identification de l'axe d'action de l'outil, dans le cas où l'outil est un système d'analyse monté sur un support placé dans l'enceinte opératoire mais indépendant du support du premier dispositif, est réalisée en visant la mire par le premier dispositif et par ledit outil.

6. Procédé selon la revendication 5, dans lequel l'outil est un appareil de radiothérapie dont la sphère d'action est matérialisée par des faisceaux laser, et dans lequel à la mire est adjoint un réflecteur cubique positionné de façon déterminée par rapport auxdits trois autres fils, et en ce que ce réflecteur cubique est placé initialement dans ladite sphère d'action en alignant les faisceaux laser sur les arêtes du cube.

7. Procédé de détermination de la position d'un organe selon la revendication 1, dans lequel :
un troisième dispositif de formation d'image tel qu'un appareil de type caméra gamma-scintigraphique, TEP, MEG ou à rayonnement synchrotron est placé dans la même enceinte opératoire que le premier dispositif,
les positions relatives du premier dispositif et du troisième sont déterminées en visant la mire qui est liée au support du patient par le premier dispositif et le troisième, et
une fusion d'image est réalisée entre les images du premier dispositif et du troisième.

8. Procédé selon la revendication 7, dans lequel le troisième dispositif est un appareil du type caméra de gamma-scintigraphie ou MEG, caractérisé en ce que ladite mire est constituée en outre de tubes creux remplis d'un produit radioactif, la mire comprenant quatre tubes montés non parallèlement les uns aux autres entre deux plaques parallèles.

## Claims

1. A method for determining the position of a patient's organ with respect to at least two imaging devices, wherein:
the first imaging device is an echography probe disposed in an operation site associated with a first coordinate system, and provides an image of points of the surface of the organ or of a skin region of the patient,
the second imaging device is disposed in a pre-operation site associated with a second coordinate system, and provides a 3D image of the surface of the organ, and, if required, of the skin surface,
this method including the following steps:
determining the position of the first device with respect to the first coordinate system;
making at least one cliché with the first device, for obtaining a first image corresponding to a cloud of points of the surface of the organ or of the skin region;
making, as a second image, at least one 3D image of the organ and of its surface or of the surface of the skin region with the second imaging device and identifying the shape of this surface to obtain a reference; and
matching the first and second images by using said reference whereby the first coordinate system is matched with the second coordinate system;
wherein the step of determining the position of the first device with respect to the first coordinate system includes the steps consisting in:
- fixing the probe onto an articulated arm of a coded robot,
- pinpointing three points of an echography sighting mark, formed by three parallel threads (1-3) tightened between two planes, and by three additional threads (4-6) that are made of a material reflecting ultra-sonic frequencies, and arranged in a triangle abutting against the three parallel threads, the set of threads being immersed in a medium capable of transmitting ultra-sonic frequencies, such as a water vessel,
- rotating the probe by 180° about its pinpoint axis and pinpointing again the three points of the sighting mark, and
- resuming the two preceding steps for another position of the sighting mark.

2. The method of claim 1, wherein the first coordinate system is the coordinate system of the patient's support.

3. The method of claim 1, wherein the echography probe is capable of providing, at each actuation, the distances within a viewing plane between said probe and the interface within said plane between said organ and the adjacent medium, and wherein the imaging technique consists in moving the probe and making a view for each of a plurality of predetermined positions of the probe so as to obtain, for each view, the position with respect to the probe of at least one point of the surface of the organ, whereby a first image corresponding to a cloud of points of the surface of the organ is obtained.

4. A method for determining the position of a patient's organ according to claim 1, characterized in that it consists in:
- determining an action axis for an operation tool with respect to the pre-operation image taken in the second coordinate system,
- identifying said action axis in the first coordinate system, and
- positioning a tool according to said action axis in the first coordinate system.

5. The method of claim 4, wherein the identification of the action axis of the tool, when said tool is an analysis system mounted on a support placed in the operation site but independent of the support of the first device, is made by pinpointing the sighting mark by the first device and by said tool.

6. The method of claim 5, wherein said tool is a radiotherapy apparatus having its sphere of action materialized by laser beams, wherein a cubic reflector positioned in a determined manner with respect to said three additional threads is added to the sighting mark, and wherein said cubic reflector is initially placed in said sphere of action by aligning the laser beams with the edges of the cube.

7. The method of claim 1, wherein:
a third imaging device such as a gamma-scintigraphy, PET, MEG or synchrotron radiation apparatus, is placed in the same operation site as the first device,
the relative positions of the first and third devices are defined by viewing the sighting mark which is fixed to the patient's support with the first and third devices, and
an image matching is made between the images of the first and third devices.

8. The method of claim 7, wherein the third device belongs to the group comprising a gamma-scintigraphy camera and a MEG apparatus, the sighting mark being further formed by hollow catheters filled with a radioactive product, the sighting mark including four tubes arranged in a noncollinear manner between two parallel plates.

## Patentansprüche

1. Verfahren zur Bestimmung der Position eines Organs eines Patienten in Bezug zu mindestens zwei bilderzeugenden Vorrichtungen, wobei:
die erste bilderzeugende Vorrichtung in einem, einem ersten Bezugssystem zugeordneten Operationsraum gelegen ist und ein Punktebild der Oberfläche des Organs oder eines Hautbereiches des Patienten liefert,
die zweite bilderzeugende Vorrichtung in einem einem zweiten Bezugssystem zugeordneten Präoperationsraum gelegen ist und ein dreidimensionales Bild der Oberfläche des Organs und gegebenenfalls der Hautoberfläche liefert,
wobei das Verfahren die folgenden Schritte aufweist:
Bestimmen der Position der ersten Vorrichtung in Bezug zu dem ersten Bezugssystem;
Machen zumindest einer Aufnahme mit der ersten Vorrichtung, um ein erstes Bild entsprechend einer Punktewolke der Oberfläche des Organs oder des Hautbereiches zu erhalten;
Aufnehmen zumindest eines dreidimensionalen Bildes als zweites Bild des Organs und seiner Oberfläche oder der Oberfläche des Hautbereiches mit Hilfe der zweiten Vorrichtung und Identifizieren der Form dieser Oberfläche, um eine Referenz zu erhalten; und
Vereinigen des ersten und zweiten Bildes unter Verwendung der Referenz, wodurch das erste Bezugssystem in Bezug zu dem zweiten markiert wird;
wobei der Schritt zum Bestimmen der Position der ersten Vorrichtung in Bezug zu dem ersten Bezugssystem folgende Schritte aufweist:
- Befestigen der Sonde auf einem codiert verschwenkbaren Robotarm,
- Anvisieren dreier Punkte eines echographischen Testobjektes, das aus drei zwischen zwei Ebenen gespannten parallelen Drähten (1 - 3)und drei weiteren Drähten (4 - 6) aus Ultraschall reflektierendem Material gebildet ist, die in einem Dreieck zwischen den drei parallelen Drähten befestigt sind, wobei das Ensemble in ein Ultraschall hindurch lassendes Medium, zum Beispiel einen Wasserbehälter, eingetaucht ist,
- Drehen der Sonde um 180° um deren Visierlinie und nochmaliges Anvisieren der drei Punkte des Testobjektes, und
- Wiederholen der zwei vorhergehenden Schritte für eine andere Position des Testobjektes.

2. Verfahren nach Anspruch 1, dadurch gekennzeichnet, dass das erste Bezugssystem das Bezugssystem der Liege des Patienten ist.

3. Verfahren zur Bestimmung der Position eines Organs nach Anspruch 1, dadurch gekennzeichnet, dass die echographische Sonde in der Lage ist, bei jeder Aufnahme die Entfernungen in einer Visierebene zwischen dieser Sonde und der Grenzfläche in dieser Ebene zwischen dem Organ und dem angrenzenden Medium zu liefern, und dass der Aufnahmeschritt darin besteht, die Sonde zu verschieben und für jede der mehreren bestimmten Lagen dieser Sonde eine Aufnahme zu machen, um dadurch für jede Aufnahme die Position zumindest eines Punktes der Oberfläche des Organs in Bezug zu der Sonde zu erhalten, wodurch ein erstes Bild entsprechend einer Punktewolke der Oberfläche des Organs erhalten wird.

4. Verfahren zur Bestimmung der Position eines Organs nach Anspruch 1, dadurch gekennzeichnet, dass es besteht aus:
- Bestimmen einer Aktionsachse eines Operationsinstrumentes in Bezug zu dem präoperativem Bild, das in dem zweiten Bezugssystem aufgenommen wurde,
- Identifizieren dieser Aktionsachse in dem erstem Bezugssystem, und
- Positionieren eines Instrumentes längs dieser Aktionsachse in dem ersten Bezugssystem.

5. Verfahren nach Anspruch 4, wobei im Falle, dass das Instrument ein Analysesystem ist, das auf einem Träger in dem Operationsraum montiert, jedoch unabhängig von dem Träger der ersten Vorrichtung ist, das Identifizieren der Aktionsachse realisiert wird, indem das Testobjekt durch die erste Vorrichtung und durch das Instrument anvisiert wird.

6. Verfahren nach Anspruch 5, bei dem das Instrument ein Strahlentherapiegerät ist, dessen Aktionssphäre durch Laserstrahlen dargestellt ist, und bei dem dem Testobjekt ein kubischer Reflektor hinzu gefügt ist, der determiniert in Bezug zu den drei weiteren Drähten positioniert ist, und wobei dieser kubische Reflektor anfänglich in der Aktionsshäre platziert wird, indem die Laserstrahlen längs der Ränder des Kubus ausgerichtet werden.

7. Verfahren zur Bestimmung der Position eines Organs nach Anspruch 1, wobei:
eine dritte bilderzeugende Vorrichtung, zum Beispiel eine Gamma-Scintigraphie-Kamera, ein TEP-, MEG- oder Synchrotron-Strahlungs-Gerät, in dem gleichen Operationsraum wie die erste Vorrichtung angeordnet wird,
die relativen Positionen der ersten und der dritten Vorrichtung durch Anvisieren des auf der Liege des Patienten gelegenen Testobjektes durch die erste und die dritte Vorrichtung bestimmt werden, und
die Bilder der ersten und der dritten Vorrichtung vereinigt werden.

8. Verfahren nach Anspruch 7, wobei die dritte Vorrichtung ein Gerät nach Art einer Gamma-Scintigraphie-Kamera oder ein MEG-Gerät ist, dadurch gekennzeichnet, dass das Testobjekt weiterhin Hohlrohre aufweist, die mit einem radioaktiven Stoff gefüllt sind, wobei das Testobjekt vier Hohlrohre aufweist, die zueinander nicht parallel zwischen zwei parallelen Platten montiert sind.
